# EUROPÄISCHE PATENTSCHRIFT

(11) **EP 0 695 302 B1**
(45) Veröffentlichungstag und Bekanntmachung des Hinweises auf die Patenterteilung: **13.08.1997**
(21) Anmeldenummer: 94913587.5
(22) Anmeldetag: 13.04.1994
(51) Int. Cl.: C07D 409/12, C07D 405/12, C07D 401/12, A01N 43/54, A01N 43/66, C07D 333/24, C07D 213/55

(54) **3-HALOGEN-3-HETARYLCARBONSÄUREDERIVATE, VERFAHREN UND ZWISCHENPRODUKTE ZU IHRER HERSTELLUNG**
3-HALO-3-HETEROARYL CARBOXYLIC ACID DERIVATIVES, METHODS OF PREPARING THEM AND INTERMEDIATES USED IN THEIR PREPARATION
DERIVES DE L'ACIDE 3-HALOGENO-3-HETARYLCARBOXYLIQUE, PROCEDE ET PRODUITS INTERMEDIAIRES POUR LEUR FABRICATION

(30) Priorität: 23.04.1993 DE 4313411
(43) Veröffentlichungstag der Anmeldung: 07.02.1996
(73) Patentinhaber: BASF AKTIENGESELLSCHAFT, 67056 Ludwigshafen (DE)
(72) Erfinder: BAUMANN, Ernst, D-67373 Dudenhofen (DE); HARREUS, Albrecht, D-67063 Ludwigshafen (DE); BRATZ, Matthias, D-67346 Speyer (DE); RHEINHEIMER, Joachim, D-67063 Ludwigshafen (DE); VOGELBACHER, Uwe, Josef, D-67071 Ludwigshafen (DE); THEOBALD, Hans, D-67117 Limburgerhof (DE); GERBER, Matthias, D-67117 Limburgerhof (DE); WALTER, Helmut, D-67283 Obrigheim (DE); WESTPHALEN, Karl-Otto, D-67346 Speyer (DE); RADEMACHER, Wilhelm, D-67117 Limburgerhof (DE)
(86) Internationale Anmeldenummer: EP9401140
(87) Internationale Veröffentlichungsnummer: WO9425455

(56) Entgegenhaltungen:
- EP-A- 0 347 811
- EP-A- 0 409 368
- EP-A- 0 517 215
- DE-A- 4 035 758
- FR-A- 2 466 451

## Beschreibung

Die vorliegende Erfindung betrifft 3-Halogen-3-hetarylcarbonsäurederivate der allgemeinen Formel I, 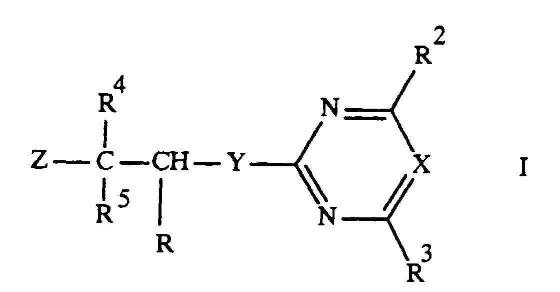 in der R eine Formylgruppe, eine Gruppe CO₂H oder einen zu COOH hydrolysierbaren Rest bedeutet und die übrigen Substituenten folgende Bedeutung haben:
- R²: Halogen, C₁-C₄-Alkyl, C₁-C₄-Halogenalkyl, C₁-C₄-Alkoxy, C₁-C₄-Halogenalkoxy oder C₁-C₄-Alkylthio;
- X: Stickstoff oder CR¹³, wobei R¹³ Wasserstoff bedeutet oder zusammen mit R³ eine 3- bis 4-gliedrige Alkylen- oder Alkenylenkette bildet, in der jeweils eine Methylengruppe durch Sauerstoff ersetzt ist;
- R³: Halogen, C₁-C₄-Alkyl, C₁-C₄-Halogenalkyl, C₁-C₄-Alkoxy, C₁-C₄-Halogenalkoxy, C₁-C₄-Alkylthio oder R³ ist mit R¹³ wie oben angegeben zu einem 5- oder 6-gliedrigen Ring verknüpft;
- R⁴: ein fünf- oder sechsgliedriger Heteroaromat, enthaltend ein bis drei Stickstoffatome und/oder ein Schwefel- oder Sauerstoffatom, welcher einen oder mehrere der folgenden Reste tragen kann: Halogen, Nitro, Cyano, Hydroxy, Mercapto, Amino, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino, Di-C₁-C₄-alkylamino, C₁-C₄-Alkylcarbonyl, C₁-C₄-Alkoxycarbonyl oder Phenyl;
- R⁵: Wasserstoff, C₁-C₄-Alkyl, C₃-C₆-Alkenyl, C₃-C₆-Alkinyl, C₃-C₈-Cycloalkyl, C₁-C₄-Halogenalkyl, C₁-C₄-Alkoxyalkyl, C₁-C₄-Alkylthioalkyl oder Phenyl;
- Y: Schwefel oder Sauerstoff oder eine Einfachbindung;
- Z: Halogen.

In der Literatur, z.B. EP-A 347 811, EP-A 400 741, EP-A 409 368, EP-A 481 512, EP-A 517 215, DE-A-4 035 758 und der älteren deutschen Anmeldung P 41 42 570 (EP-A-548 710) werden ähnliche Carbonsäurederivate, unter anderem auch 3-Halogenderivate beschrieben, jedoch keine, die einen Hetaryl-Rest in 3-Stellung tragen. Aus FR-A-2 466 451 sind weiter Halogen-3-thienyl- bzw. Halogen-3-furylcarbonsäurederivate bekannt, die als Vorstufe zur Herstellung entsprechender erfindungsgemäßer 3-Halogen-3-hetarylcarbonsäurederivate I verwendet werden können.

Da die biologische Wirkung und Selektivität der bekannten Verbindungen nicht immer befriedigend ist, lag der Erfindung die Aufgabe zugrunde, Verbindungen mit verbesserter Selektivität gegenüber Kulturpflanzen und/oder besserer herbizider oder bioregulatorischer Wirkung bereitzustellen.

Es wurde nun gefunden, daß die eingangs definierten 3-Halogen-3-hetaryl-carbonsäurederivate ausgezeichnete herbizide und pflanzenwachstumsregulierende Eigenschaften haben.

Die Herstellung der erfindungsgemäßen Verbindungen geht aus von den Epoxiden IV, die man in allgemein bekannter Weise, z.B. wie in J. March, Advanced Organic Chemistry, 2nd ed., 1983, S. 862 bzw. S. 750 beschrieben, aus den Aldehyden bzw. Ketonen II oder den Olefinen III erhält:

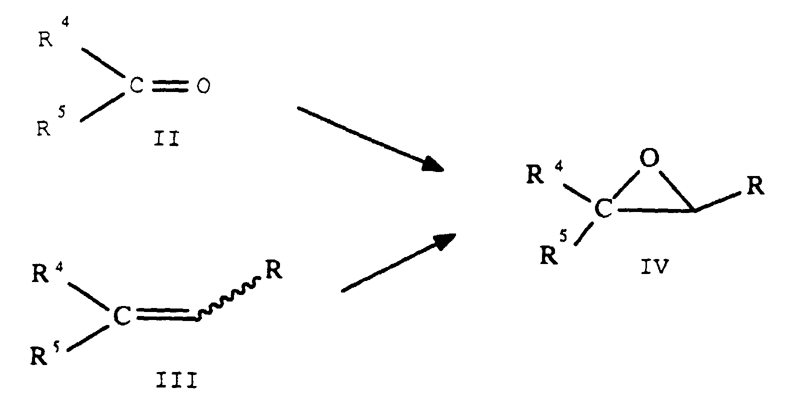

3-Halogen-3-hetaryl-carbonsäurederivate der Formel VI können hergestellt werden, indem man die Epoxide.der Formel IV (z.B. mit R = COOR⁹) mit Halogenderivaten MZ der Formel V, in der Z die in Anspruch 1 genannte Bedeutung hat und M Wasserstoff, ein Alkalimetallkation oder das Äquivalent eines Erdalkalimetallkations bedeutet, zur Reaktion bringt:

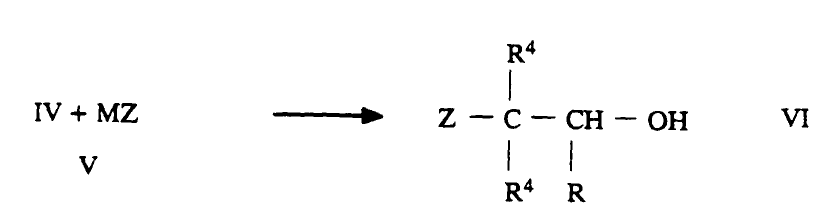

Die Reaktion kann auch in Gegenwart eines Verdünnungsmittels erfolgen. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide und Sulfone, wie zum Beispiel Dimethylsulfoxid und Sulfolan, und Basen, wie zum Beispiel Pyridin.

Die Reaktion wird dabei bevorzugt in einem Temperaturbereich zwischen 0°C und dem Siedepunkt des Lösungsmittels bzw. Lösungsmittelgemisches durchgeführt.

Die Gegenwart eines Reaktionskatalysators kann von Vorteil sein. Als Katalysatoren kommen dabei organische Säuren und anorganische Säuren sowie Lewissäuren in Frage. Beispiele hierfür sind unter anderem Schwefelsäure, Salzsäure, Trifluoressigsäure, Bortrifluorid-Etherat und Titan (IV) -Halogenide.

Die erfindungsgemäßen Verbindungen, in denen Y Sauerstoff bedeutet und die restlichen Substituenten, die unter der allgemeinen Formel I angegebenen Bedeutung haben, können beispielsweise derart hergestellt werden, daß man die 3-Halogen-3-hetaryl-carbonsäurederivate der allgemeinen Formel VI, mit Verbindungen der allgemeinen Formel VII, 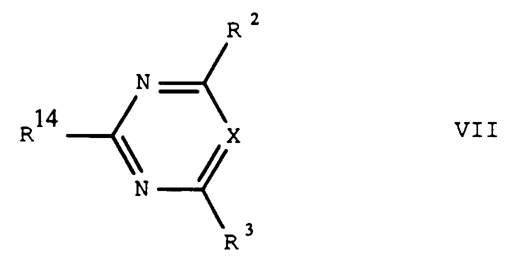 in der R¹⁴ Halogen oder R¹⁵SO₂- bedeutet, wobei R¹⁵ für C₁-C₄-Alkyl, C₁-C₄-Halogenalkyl oder Phenyl steht, zur Reaktion bringt. Die Reaktion findet bevorzugt in einem der oben genannten inerten Verdünnungsmittel unter Zusatz einer geeigneten Base, d.h. einer Base, die die Verbindung VI zu deprotonieren vermag, in einem Temperaturbereich von Raumtemperatur bis zum Siedepunkt des Lösungsmittels statt.

Als Base kann ein Alkali- oder Erdalkalimetallhydrid wie Natriumhydrid, Kaliumhydrid oder Calciumhydrid, ein Carbonat, z.B. Alkalimetallcarbonat wie Natrium- oder Kaliumcarbonat, ein Alkalimetallhydroxid wie Natrium- oder Kaliumhydroxid, eine metallorganische Verbindung wie Butyllithium oder ein Alkaliamid wie Lithiumdiisopropylamid dienen.

Die erfindungsgemäßen Verbindungen, in denen Y Schwefel bedeutet und die restlichen Substituenten die unter der Formel I angegebene Bedeutung haben, können beispielsweise derart hergestellt werden, daß man 3-Halogen-3-hetaryl-carbonsäurederivate der allgemeinen Formel VIII, 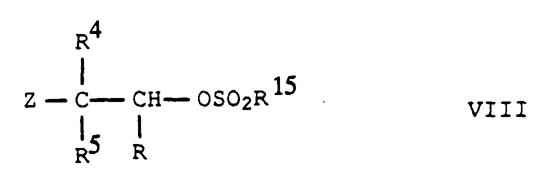 die in bekannter Weise aus Verbindungen der allgemeinen Formel VI erhältlich sind und in denen die Substituenten die oben angegebene Bedeutung haben, mit Verbindungen der allgemeinen Formel IX, 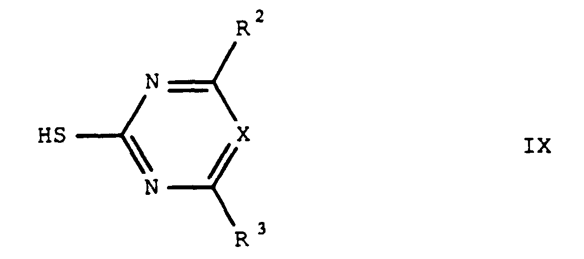 in der R², R³ und X die unter der allgemeinen Formel I angegebene Bedeutung haben, zur Reaktion bringt.

Die Reaktion findet bevorzugt in einem der oben genannten inerten Verdünnungsmittel unter Zusatz einer geeigneten Base, d.h. einer Base, die das Zwischenprodukt IX zu deprotonieren vermag, in einem Temperaturbereich von Raumtemperatur bis zum Siedepunkt des Lösungsmittels statt.

Als Base können neben den oben genannten auch organische Basen wie Triethylamin, Pyridin, Imidazol oder Diazabicycloundecan dienen.

Verbindungen der Formel I können auch dadurch hergestellt werden, daß man von den entsprechenden Carbonsäuren, d.h. Verbindungen der Formel I, in denen R COOH bedeutet, ausgeht und diese zunächst auf übliche Weise in eine aktivierte Form wie ein Halogenid, ein Anhydrid oder Imidazolid überführt und dieses dann mit einer entsprechenden Hydroxylverbindung HOR⁹ umsetzt. Diese Umsetzung läßt sich in den üblichen Lösungsmitteln durchführen und wird vorteilhaft in Gegenwart einer Base, wobei die oben genannten in Betracht kommen, vorgenommen. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Außerdem können Verbindungen der Formel I auch dadurch hergestellt werden, daß man von den Salzen der entsprechenden Carbonsäuren ausgeht, d.h. z.B. von Verbindungen der Formel I, in denen R für eine Gruppe COR und R¹ für OM stehen, wobei M z.B. ein Alkalimetallkation oder das Äquivalent eines Erdalkalimetallkations sein kann. Diese Salze lassen sich mit vielen Verbindungen der Formel R¹-A zur Reaktion bringen, wobei A eine übliche nucleofuge Abgangsgruppe bedeutet, beispielsweise Halogen wie Chlor, Brom, Iod oder gegebenenfalls durch Halogen, Alkyl oder Halogenalkyl substituiertes Aryl- oder Alkylsulfonyl wie z.B. Toluolsulfonyl und Methylsulfonyl oder eine andere äquivalente Abgangsgruppe. Verbindungen der Formel R¹-A mit einem reaktionsfähigen Substituenten A sind bekannt oder nach allgemeinem Fachwissen leicht zugänglich. Diese Umsetzung läßt sich in den üblichen Lösungsmitteln durchführen und wird vorteilhaft in Gegenwart einer Base, wobei die oben genannten in Betracht kommen, vorgenommen.

Der Rest R in Formel I ist breit variabel. Beispielsweise steht R für eine Gruppe 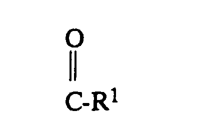 in der R¹ die folgende Bedeutung hat:
a) Wasserstoff;
b) eine Succinylimidoxygruppe;
c) ein über ein Stickstoffatom verknüpfter 5-gliedriger Heteroaromat wie Pyrrolyl, Pyrazolyl, Imidazolyl und Triazolyl, welcher ein bis zwei Halogenatome, insbesondere Fluor und Chlor und/oder ein bis zwei der folgenden Reste tragen kann:
   C₁-C₄-Alkyl wie Methyl, Ethyl, 1-Propyl, 2-Propyl, 2-Methyl-2-propyl, 2-Methyl-1-propyl, 1-Butyl, 2-Butyl;
   C₁-C₄-Halogenalkyl, insbesondere C₁-C₂-Halogenalkyl wie beispielsweise Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;
   C₁-C₄-Ralogenalkoxy, insbesondere C₁-C₂-Halogenalkoxy wie Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;
   C₁-C₄-Alkoxy wie Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
   C₁-C₄-Alkylthio wie Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
d) R¹ ferner ein Rest -(O)ₘ-NR⁶R⁷ in dem m für 0 oder 1 steht und R⁶ und R⁷, die gleich oder unterschiedlich sein können, die folgende Bedeutung haben:
   Wasserstoff
   C₃-C₈-Alkyl, insbesondere C₁-C₄-Alkyl wie oben genannt;
   C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;
   C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl;
   C₃-C₈-Cycloalkyl, wie Cyclopropyl, Cyclobutyl, Cyclopentyl, Cyclohexyl, Cyclohepthyl und Cycclooctyl, wobei diese Alkyl-, Cycloalkyl-, Alkenyl- und Alkinylgruppen jeweils ein bis fünf, insesondere ein bis drei Halogenatome, bevorzugt Fluor oder Chlor und/oder ein bis zwei der folgenden Gruppen tragen können:
   C₁-C₄-Alkyl, C₁-C₄-Alkoxy, C₁-C₄-Alkylthio, C₁-C₄-Halogenalkoxy wie vorstehend genannt, C₃-C₆-Alkenyloxy, C₃-C₆-Alkenylthio, C₃-C₆-Alkinyloxy, C₃-C₆-Alkinylthio, wobei die in diesen Resten vorliegenden Alkenyl- und Alkinylbestandteile vorzugsweise den oben genannten Bedeutungen entsprechen;
   C₁-C₄-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl;
   C₁-C₄-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl;
   C₃-C₆-Alkenylcarbonyl, C₃-C₆-Alkinylcarbonyl, C₃-C₆-Alkenyloxycarbonyl und C₃-C₆-Alkinyloxycarbonyl, wobei die Alkenyl- bzw. Alkinylreste vorzugsweise, wie voranstehend im einzelnen aufgeführt, definiert sind;
   Phenyl, gegebenenfalls ein oder mehrfach substituiert durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio wie beispielsweise 2-Fluorphenyl, 3-Chlorphenyl, 4-Bromphenyl, 2-Methylphenyl, 3-Nitrophenyl, 4-Cyanophenyl, 2-Trifluormethylphenyl, 3-Methoxyphenyl, 4-Trifluorethoxyphenyl, 2-Methylthiophenyl, 2,4-Dichlorphenyl, 2-Methoxy-3-methylphenyl, 2,4-Dimethoxyphenyl, 2-Nitro-5-cyanophenyl, 2,6-Difluorphenyl;
   Di-C₁-C₄-alkylamino wie insbesondere Dimethylamino, Dipropylamino, N-Propyl-N-methylamino, N-Propyl-N-ethylamino, Diisopropylamino, N-Isopropyl-N-methylamino, N-Isopropyl-N-ethylamino, N-Isopropyl-N-propylamino;
   R⁶ und R⁷ ferner Phenyl, das durch einen oder mehrere der folgenden Reste substituiert sein kann: Halogen, Nitro, Cyano, C₁-C₄-Alkyl, C₁-C₄-Ralogenalkyl, C₁-C₄-Alkoxy, C₁-C₄-Halogenalkoxy oder C₁-C₄-Alkylthio, wie im einzelnen oben genannt;
   oder R⁶ und R⁷ bilden gemeinsam eine zu einem Ring geschlossene, optionell substituierte C₄-C₇-Alkylenkette, die ein Heteroatom, ausgewählt aus der Gruppe Sauerstoff, Schwefel oder Stickstoff, enthalten kann wie -(CH₂)₄-, -(CH₂)₅-, -(CH₂)₆-, -(CH₂)₇-, -(CH₂)₂-O-(CH₂)₂-, -CH₂-S-(CH₂)₃-, -(CH₂)₂-O-(CH₂)₃-, -NH-(CH₂)₃-, -CH₂-NH-(CH₂)₂-, -CH₂-CH=CH-CH₂-, -CH=CH-(CH₂)₃-, wobei als Substituenten insbesondere C₁-C₄-Alkylreste in Betracht kommen;
e) R¹ ferner eine Gruppe 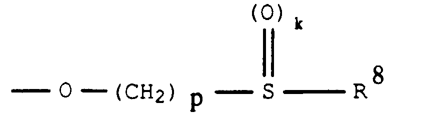 in der k die Werte 0, 1 und 2, p die Werte 1, 2, 3 und 4 annehmen und R⁸ für
   C₁-C₄-Alkyl, C₁-C₄-Ralogenalkyl, C₃-C₆-Alkenyl, C₃-C₆-Alkinyl oder gegebenenfalls substituiertes Phenyl steht, wie insbesondere für R⁶ und R⁷ genannt;
f) R¹ ferner ein Rest OR⁹, worin R⁹ bedeutet:
   i) Wasserstoff, das Kation eines Alkalimetalls oder das Kation eines Erdalkalimetalls wie Lithium, Natrium, Kalium, Calcium, Magnesium und Barium oder ein umweltverträgliches organisches Ammoniumion wie tert,-C₁-C₄-Alkylammonium oder Ammonium [NH₄⁺];
   ii) C₃-C₈-Cycloalkyl wie vorstehend genannt, welches ein bis drei C₁-C₄-Alkylgruppen tragen kann;
   iii) C₁-C₈-Alkyl wie insbesondere Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, welches ein bis fünf Halogenatome, insbesondere Fluor und Chlor und/ oder einen der folgenden Reste tragen kann:
      C₁-C₄-Alkoxy, C₁-C₄-Alkylthio, Cyano, C₁-C₄-Alkylcarbonyl, C₃-C₈-Cycloakyl, C₁-C₄-Alkoxycarbonyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy und/ oder C₁-C₄-Alkylthio substituiertes Phenyl oder Phenoxy, wie insbesondere oben genannt;
   iv) eine C₁-C₈-Alkylgruppe wie vorstehend genannt, welche ein bis fünf, vorzugsweise ein bis drei Halogenatome, insbesondere Fluor und/oder Chlor tragen kann und einen der folgenden Reste trägt: ein 5-gliedriger Heteroaromat, enthaltend ein bis drei Stickstoffatome oder ein 5-gliedriger Heteroaromat, enthaltend ein Stickstoffatom und ein Sauerstoff- oder Schwefelatom, wie Pyrazolyl, Imidazolyl, Benzimidazolyl, Triazolyl, Benztriazolyl, Isooxazolyl, Oxazolyl, Thiazolyl, gebunden über ein C-Atom oder falls möglich N-Atom, wobei der Heteroaromat ein bis vier Halogenatome und/oder ein bis zwei der folgenden Reste tragen kann:
      Nitro, Cyano, C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl,.4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-tert.-Butylisoxazol-5-yl;
   v) eine C₂-C₆-Alkylgrupe, welche in der 2-Position einen der folgenden Reste trägt: C₁-C₄-Alkoxyimino, C₃-C₆-Alkinyloxyimino, C₃-C₆-Ralogenalkenyloxyimino oder Benzyloxyimino;
   vi) eine C₃-C₆-Alkenyl- oder eine C₃-C₆-Alkinylgruppe, wobei diese Gruppen ihrerseits ein bis fünf Halogenatome tragen können;
   vii) R⁹ ferner ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Ralogenalkoxy und/oder C₁-C₄-Alkylthio, wie insbesondere oben genannt;
   viii) ein über ein Stickstoffatom verknüpfter 5-gliedriger Heteroaromat, enthaltend ein bis drei Stickstoffatome wie Pyrazolyl, Imidazolyl, Benzimidazolyl, Triazolyl, Benztriazolyl, vorzugsweise gebunden über die 1-Position, wobei der Heteroaromat ein bis zwei Halogenatome und/oder ein bis zwei der folgenden Reste tragen kann:
      C₁-C₄-Alkyl, C₁-C₄-Ralogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3,4-Dichlorimidazol-l-yl;
   ix) R⁹ ferner ein Gruppe 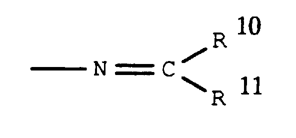 worin R¹⁰ und R¹¹, die gleich oder verschieden sein können, bedeuten:
      C₁-C₈-Alkyl, C₃-C₆-Alkenyl, C₃-C₆-Alkinyl, C₃-C₈-Cycloalkyl, wobei diese Reste einen C₁-C₄-Alkoxy, C₁-C₄-Alkylthio und/oder einen gegebenenfalls substituierten Phenylrest, wie insbesondere vorstehend genannt, tragen können;
      Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio, wobei diese Reste insbesondere den oben für R¹ genannten entsprechen;
      oder R¹⁰ und R¹¹ bilden gemeinsam eine C₃-C₁₂-Alkylenkette, welche ein bis drei C₁-C₄-Alkylgruppen tragen und ein Heteroatom aus der Gruppe Sauerstoff, Schwefel und Stickstoff enthalten kann, wie insbesondere bei R⁶ und R⁷ genannt.
g) R¹ ferner ein Rest -NH-SO₂-R¹² worin R¹² bedeutet:
   C₁-C₄-Alkyl, C₃-C₆-Alkenyl, C₃-C₆-Alkinyl, C₃-C₈-Cycloalkyl wie insbesondere vorstehend für R¹ genannt, wobei diese Reste einen C₁-C₄-Alkoxy-, C₁-C₄-Alkylthio- und/oder einen Phenylrest wie oben genannt tragen können;
   Phenyl, gegebenenfalls substituiert, insbesondere wie vorstehend genannt.

Im Hinblick auf die biologische Wirkung sind 3-Halogen-3-hetarylcarbonsäurederivate der Formel I bevorzugt, in der die übrigen. Substituenten folgende Bedeutung haben:
- R²: die bei R¹ im einzelnen genannten C₁-C₄-Alkyl-, C₁-C₄-Halogenalkyl-, C₁-C₄-Alkoxy-, C₁-C₄-Halogenalkoxy-, C₁-C₄-Alkylthiogruppen und Halogenatome bedeutet, insbesondere Chlor, Methyl, Methoxy, Ethoxy, Difluormethoxy, Trifluormethoxy, besonders bevorzugt Methoxy;
- X: Stickstoff oder CR¹³, worin
- R¹³: bevorzugt Wasserstoff bedeutet oder zusammen mit R³ eine 4 - bis 5-gliedrige Alkylen- oder Alkenylenkette bildet, in der jeweils eine Methylengruppe durch Sauerstoff ersetzt ist wie -CH₂-CH₂-O-, -CH=CH-O-, -CH₂-CH₂-CH₂-O-, -CH=CH- CH₂O-, insbesondere Wasserstoff und -CH₂-CH₂-O-;
- R³: die bei R¹ genannten C₁-C₄-Alkyl-, C₁-C₄-Ralogenalkyl-, C₁-C₄-Alkoxy-, C₁-C₄-Ralogenalkoxy-, C₁-C₄-Alkylthiogruppen und Halogenatome bedeutet, insbesondere Chlor, Methyl, Methoxy, Ethoxy, Difluormethoxy, Trifluormethoxy, besonders bevorzugt Methoxy, , oder mit R¹³ wie oben genannt zu einem 5- oder 6-gliedrigen Ring verknüpft ist;
- R⁴: ein 5- oder 6-gliedriges Heteroaryl wie Furyl, Thienyl, Pyrrolyl, Pyrazolyl, Imidazolyl, Triazolyl, Isoxazolyl, Oxazolyl, Isothiazolyl, Thiazolyl, Thiadiazolyl, Pyridyl, Pyrimidinyl, Pyrazinyl, Pyridazinyl, Triazinyl, beispielsweise 2-Furyl, 3-Furyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 4-Pyrrolyl, 3-Pyrazolyl, 4-Pyrazolyl, 5-Pyrazolyl, 2-Pyridyl, 3-Pyridyl, 4-Pyridyl, Oxa-2,4-diazolyl, Oxa-3,4-diazolyl, Thia-2,4-diazolyl, Thia-3,4-diazolyl und Triazolyl, wobei die Heteroaromaten einen oder mehrere der folgenden Reste tragen können:
Halogen, Nitro, Cyano, Hydroxy, Mercapto, Amino, C₁-C₄-Alkyl, C₁-C₄-Alkoxy, C₁-C₄-Alkylthio, C₁-C₄-Halogenalkyl, C₁-C₄-Halogenalkoxy, C₁-C₄-Alkylamino, Di-C₁-C₄-alkylamino,C₁-C₄-Alkylcarbonyl, C₁-C₄-Alkoxycarbonyl und Phenyl, wie im allgemeinen und besonderen oben genannt;
- R⁵: Wasserstoff, C₁-C₄-Alkyl, C₃-C₆-Alkenyl, C₃-C₆-Alkinyl, C₃-C₈-Cycloalkyl, C₁-C₄-Halogenalkyl, C₁-C₄-Alkoxyalkyl, C₁-C₄-Alkylthioalkyl oder Phenyl wie vorstehend genannt;
- Y: Schwefel, Sauerstoff oder eine Einfachbindung;
- Z: Halogen.

Bevorzugt sind Verbindungen I mit R⁵ = Methyl, X = CH, R² und R³ = Methoxy. Ferner Verbindungen I mit R⁵ = Methyl, X = CH, Z = Fluor und R², R³ = Methoxy. Darüber hinaus steht R¹ bevorzugt für eine Gruppe OR⁹, insbesondere OH und OC₁-C₄-Alkyl.

Die Variable Y ist bevorzugt Schwefel und insbesondere Sauerstoff.

Besonders bevorzugte Verbindungen der Formel I sind in der nachfolgenden Tabelle I aufgeführt. Die darin und in den Tabellen 1 und 2 für R⁴ aufgeführten Definitionen sind ebenfalls als bevorzugt anzusehen, unabhängig von den weiteren Restedefinitionen.

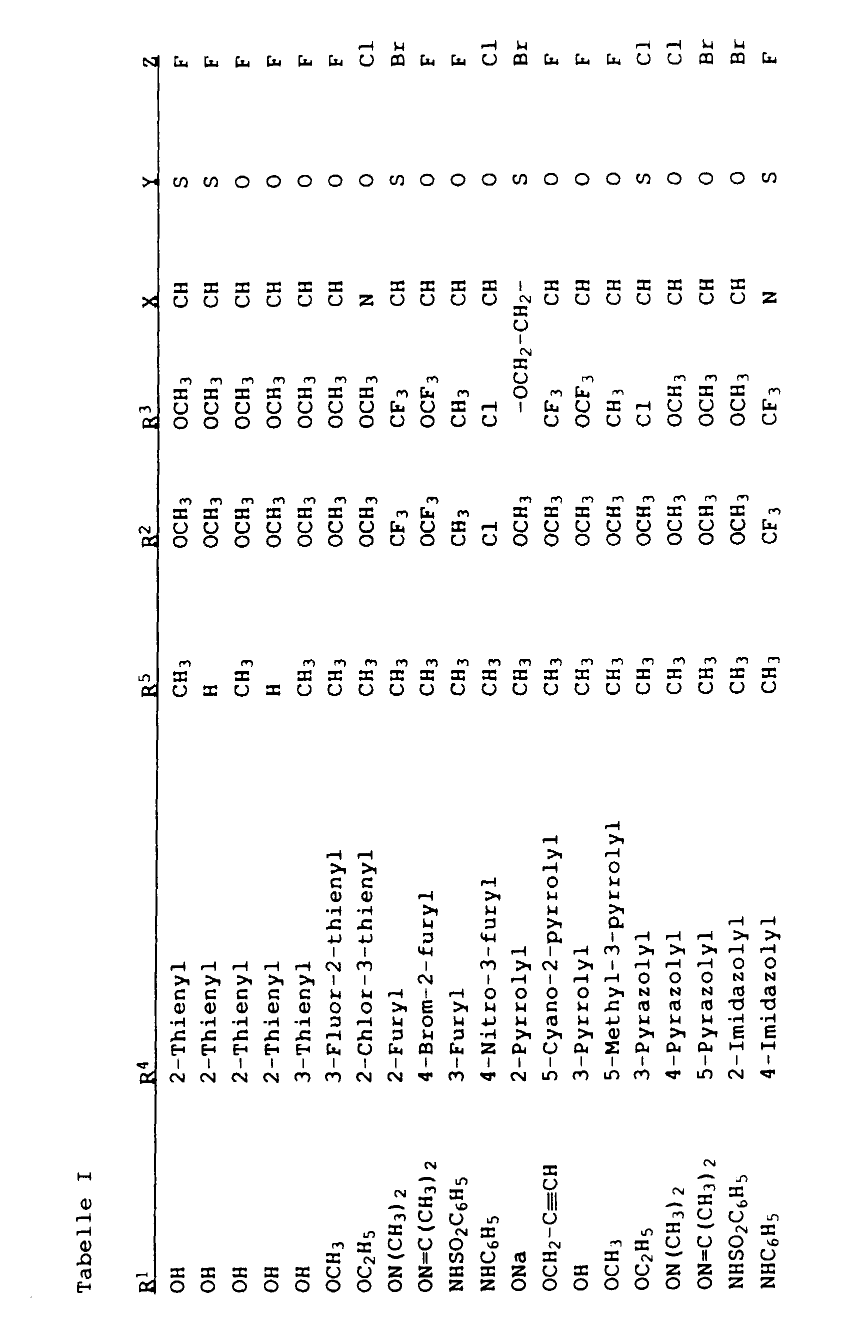

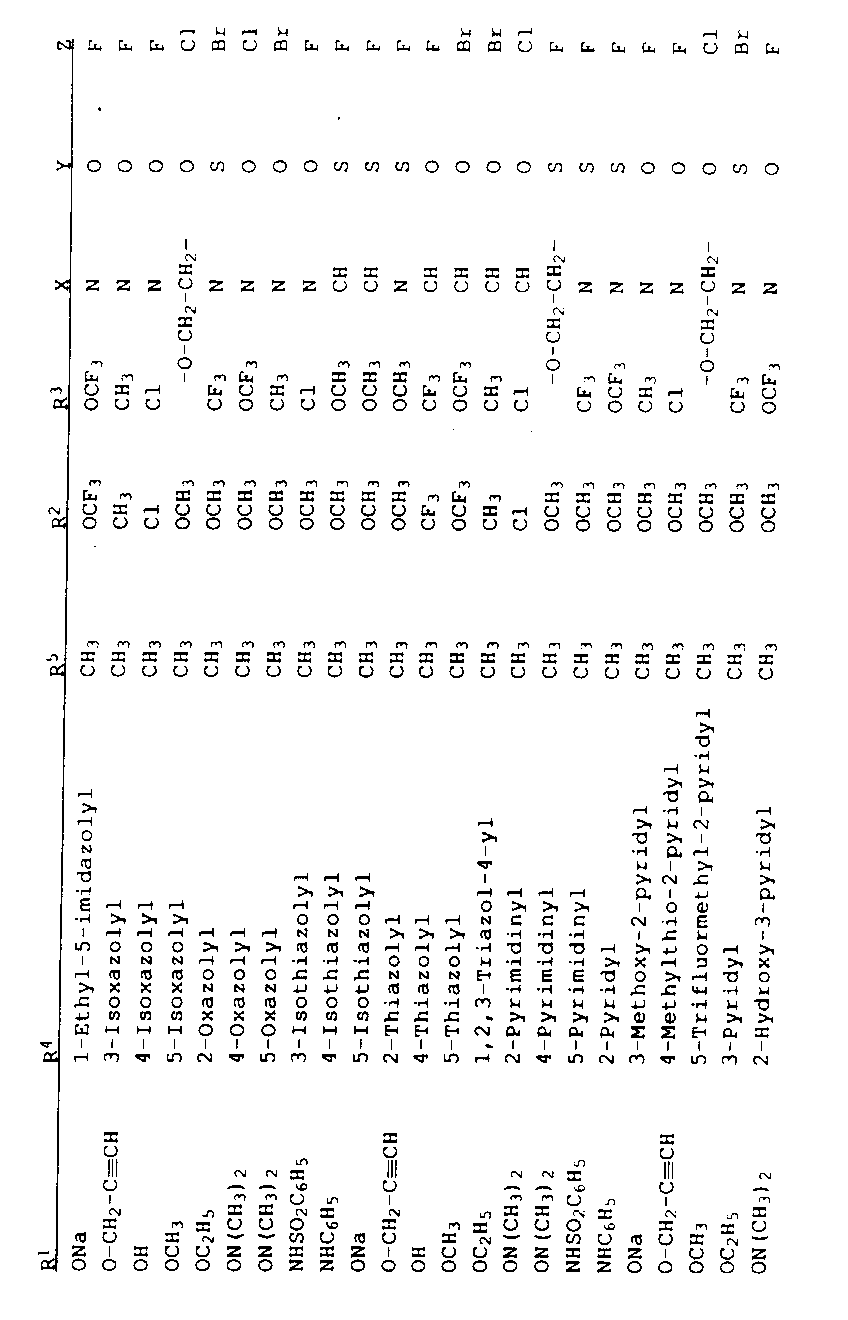

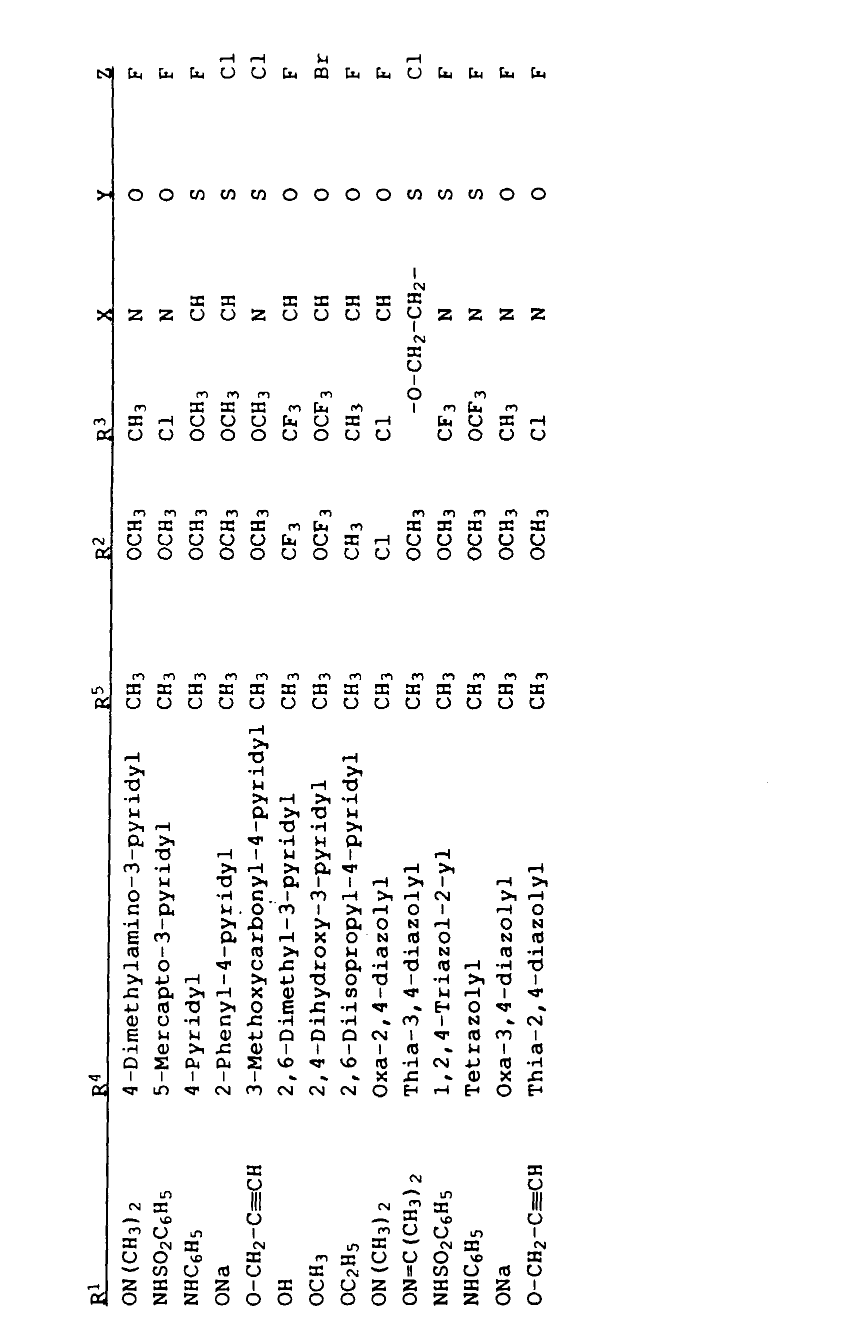

Die Verbindungen I bzw. die sie enthaltenden herbiziden Mittel sowie deren umweltverträgliche Salze, z.B. von Alkalimetallen und Erdalkalimetallen, können in Kulturen wie Weizen, Reis und Mais, Soja und Baumwolle, Unkräuter und Schadgräser sehr gut bekämpfen, ohne die Kulturpflanzen zu schädigen, ein Effekt, der vor allem auch bei niedrigen Aufwandmengen auftritt. Sie können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen u.a. Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 bis 100 %, vorzugsweise 95 bis 100 % (nach NMR-Spektrum) eingesetzt.

Beispiele für Formulierungen sind:
I. 20 Gewichtsteile der Verbindung Nr. 2.17 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
II. 20 Gewichtsteile der Verbindung Nr. 2.1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethykenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
III. 20 Gewichtsteile des Wirkstoffs Nr. 2.17 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 2.2 werden mit 3 Gewichtsteilen des Natriumsalzes der Diisobutylnaphthalinα-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
V. 3 Gewichtsteile des Wirkstoffs Nr. 2.17 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. 2.17 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5 kg/ha, vorzugsweise 0,01 bis 2 kg/ha aktive Substanz.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora,, Coffea liberica), Cucumis sativus, Cynodon dactylon, Daucus carota, Elaeis guineensis, Fragaria vesca, Glycine max, Gossypium hirsutum, (Gossypium arboreum, Gossypium, herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (S. vulgare), Theobroma cacao, Trifolium pratense, Triticum aestivum, Triticum durum, Vicia faba, Vitis vinifera, Zea mays.

Die Verbindungen der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem
a) von der Pflanzenart und -sorte,
b) vom Zeitpunkt der Applikation, bezogen auf das Entwicklungsstadium der Pflanze und von der Jahreszeit,
c) von dem Applikationsort und -verfahren (z.B. Samenbeize, Bodenbehandlung, Blattapplikation oder Stamminjektion bei Bäumen),
d) von klimatischen Faktoren, z.B. Temperatur, Niederschlagsmenge, außerdem auch Tageslänge und Lichtintensität,
e) von der Bodenbeschaffenheit (einschließlich Düngung),
f) von der Formulierung bzw. Anwendungsform des Wirkstoffs und schließlich
g) von der angewendeten Konzentration der aktiven Substanz.

Aus der Reihe der verschiedenartigen Anwendungsmöglichkeiten der Pflanzenwachstumsregulatoren der Formel I im Pflanzenanbau, in der Landwirtschaft und im Gartenbau, werden einige nachstehend erwähnt.
A. Mit den erfindungsgemäß verwendbaren Verbindungen läßt sich das vegetative Wachstum der Pflanzen stark hemmen, was sich insbesondere in einer Reduzierung des Längenwachstums äußert.
   Die behandelten Pflanzen weisen demgemäß einen gedrungenen Wuchs aus; außerdem ist eine dunklere Blattfärbung zu beobachten.
   Als vorteilhaft für die Praxis erweist sich eine verminderte Intensität des Wachstums von Gräsern sowie lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Bei Obst- und anderen Bäumen lassen sich mit den Wachstumsregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide, ist es vorteilhaft, wenn die Bestände durch Behandlung mit den erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt bzw. Pflanzenmasse - dem Befall mit verschiedenen Krankheiten (z.B. Pilzkrankheit) vorgebeugt werden.
B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Citrusfrüchten zu erhöhen, den Proteingehalt in Getreide oder Soja zu steigern oder Gummibäume zum vermehrten Latexfluß zu stimulieren.
   Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Citrusfrüchten, Oliven oder bei anderen Arten und Sorten von Kern-, Stein- und Schalenobst ermöglicht wird. Derselbe Mechanismus, d.h. die Förderung der Ausbildung von Trenngewebe zwischen Frucht-, bzw. Blatt- und Sproßteil der Pflanze ist auch für ein gut kontrollierbares Entblättern von Nutzpflanzen wie beispielsweise Baumwolle wesentlich.
D. Mit Wachstumsregulatoren kann weiterhin der Wasserverbrauch von Pflanzen reduziert werden. Durch den Einsatz der erfindungsgemäßen Substanzen läßt sich die Intensität der Bewässerung reduzieren und damit eine kostengünstigere Bewirtschaftung durchführen, weil u.a.
   - die Öffnungsweite der Stomata reduziert wird,
   - eine dickere Epidermis und Cuticila ausgebildet werden,
   - die Durchwurzelung des Bodens verbessert wird und
   - das Mikroklima im Pflanzenbestand durch einen kompakterenWuchs günstig beeinflußt wird.

Besonders gut eignen sich sich Verbindungen I zur Halmverkürzung von Kulturpflanzen wie Gerste, Raps und Weizen.

Die erfindungsgemäß zu verwendenden Wirkstoffe der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Die Aufwandmenge an Wirkstoff ist infolge der hohen Pflanzenverträglichkeit nicht kritisch. Die optimale Aufwandmenge variiert je nach Bekämpfungsziel, Jahreszeit, Zielpflanzen und Wachstumsstadien.

Bei der Saatgutbehandlung werden im allgemeinen Wirkstoffmengen von 0.001 bis 50 g, vorzugsweise 0.01 bis 10 g, je Kilogramm Saatgut benötigt.

Für die Blatt- und Bodenbehandlung sind im allgemeinen Gaben von 0.001 bis 10 kg/ha, bevorzugt 0.01 bis 3 kg/ha, insbesondere 0.01 bis 0.5 kg/ha als ausreichend zu betrachten.

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy- bzw. Heteroaryloxy-phenoxypropionsäuren sowie deren Salze, Ester und Amide und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Synthesebeispiele

### Synthese von Verbindungen der allgemeinen Formel VI

### Beispiel 1

### 3-Fluor-3-(2-thienyl)-2-hydroxybuttersäuremethylester

19,5 g (100 mmol) 3-(2-Thienyl)-2,3-epoxybuttersäuremethylester werden in 50 ml getrocknetem Dichlormethan gelöst und zu einer Lösung von 100 ml Fluorwasserstoff-Pyridin-Komplex (70 % HF) in 100 ml getrocknetem Dichlormethan getropft. Nach 1 Stunde bei Raumtemperatur rührt man die Reaktionslösung in 150 ml Eiswasser ein. Die organische Phase wird mit Bicarbonatlösung und mit Wasser gewaschen, über Magnesiumsulfat getrocknet und eingeengt. Der Rückstand wird aus Petrolether unter Zusatz von wenig Essigester umkristallisiert.
Ausbeute: 17,2 g (79 %).

### Beispiel 2

### 3-Chlor-3- (3-pyridyl)-2-hydroxybuttersäuremethylester

Man löst 0,8 g (20 mmol) LiCl in 100 ml absolutem Tetrahydrofuran (THF), kühlt auf -20°C und tropft 20 ml Titantetrachlorid (1 M in Dichlormethan) zu. Nach 30 Minuten Rühren bei -20°C kühlt man auf -78°C ab und tropft 3,8 g (20 mmol) 3-(3-Pyridyl)-2,3-epoxybuttersäuremethylester in 50 ml THF zu. Nach Aufwärmen auf Raumtemperatur wird noch 6 Stunden gerührt, das Lösungsmittel abdestilliert und der Rückstand zwischen Essigester und Wasser verteilt.

Die wäßrige Phase wird mit Essigester extrahiert, die vereinigten organischen Phasen über Natriumsulfat getrocknet und eingeengt. Der Rückstand wird durch Chromatographie an Kieselgel mit n-Hexan/Essigestergemischen weiter gereinigt. Nach Abdestillieren des Lösungsmittels verbleiben 2,9 g eines schwach gelben Öls.
Ausbeute: 63 %.

Analog wurden alle in Tabelle 1 genannten Verbindungen hergestellt.

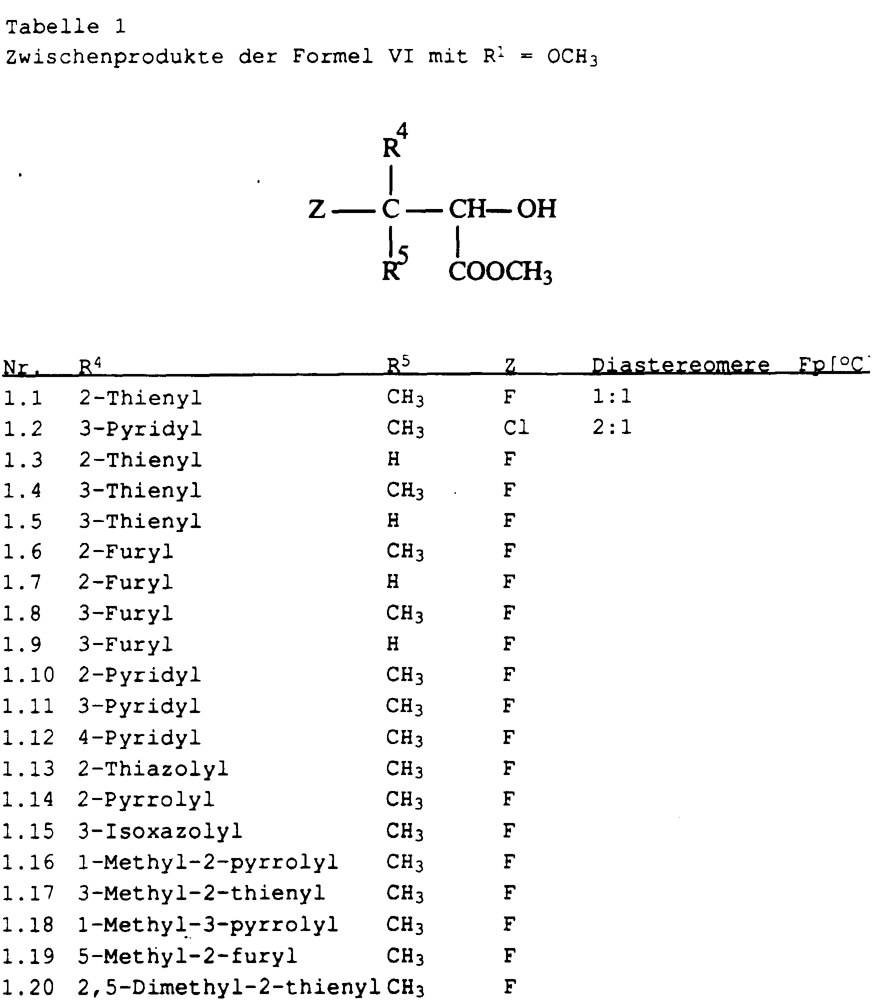

### Synthese von Verbindungen der allgemeinen Formel I:

### Beispiel 3

### 3-(2-Thienyl)-3-fluor-2-[(4,6-dimethoxypyrimidin-2-yl)oxy]buttersäuremethylester

2,2 g (10 mmol) 3-(2-Thienyl)-3-fluor-2-hydroxybuttersäuremethylester (Verb. 1.1) werden in 40 ml Dimethylformamid gelöst und mit 0,3g (12 mmol) Natriumhydrid versetzt. Man rührt 1 Stunde und gibt dann 2,2 g (10 mmol) 4,6-Dimethoxy-2-methylsulfonylpyrimidin zu. Nach 24 Stunden Rühren bei Raumtemperatur wird mit 10 ml Wasser hydrolysiert, mit Essigsäure ein pH-Wert von 5 eingestellt und das Lösungsmittel im Hochvakuum abdestilliert. Der Rückstand wird in Essigester aufgenommen, mit Wasser gewaschen, über Natriumsulfat getrocknet und das Lösungsmittel abdestilliert. Der Rückstand wird mit 10 ml Methyl-t-butylether versetzt und der gebildete Niederschlag abgesaugt. Nach dem Trocknen verbleiben 1,8 g eines weißen Pulvers.
Ausbeute: 61 % (Diastereomerengemisch 1:1).

### Beispiel 4

### 3-(2-Thienyl)-3-fluor-2-[(4,6-dimethoxypyrimidin-2-yl)oxy]buttersäure

0,9 g (3 mmol) 3-(2-Thienyl)-3-fluor-2-(4,6-dimethoxypyrimidin-2-yl)-oxybuttersäuremethylester (aus Bsp. 3) werden in 20 ml Methanol und 20 ml Tetrahydrofuran gelöst und mit 3,7 g 10 % NaOH-Lösung versetzt. Man rührt 6 Stunden bei 60°C und 12 Stunden bei Raumtemperatur, destilliert die Lösungsmittel im Vakuum ab und nimmt den Rückstand in 100 ml Wasser auf. Nun wird mit Essigester extrahiert, anschließend die Wasserphase mit verdünnter Salzsäure auf pH 1-2 eingestellt und extrahiert mit Essigester. Nach Trocknen über Magnesiumsulfat und Abdestillieren des Lösungsmittels wird der Rückstand mit wenig Aceton versetzt und der gebildete Niederschlag abgesaugt. Nach dem Trocknen verbleiben 0,8 g eines weißen Pulvers.

### Ausbeute: 89 % (Diastereomerengemisch 3:2)

### Beispiel 5

### 3-(2-Thienyl)-3-fluor-2-[(4,6-dimethoxypyrimidin-2-yl)thio]-buttersäuremethylester

5,5 g (25 mmol) 3-(2-Thienyl)-3-fluor-2-hydroxybuttersäuremethyl ester (Verb. 1.1) werden in 50 ml Dichlormethan gelöst, 3 g (30 mmol) Triethylamin zugegeben und unter Rühren 3,2 g (28 mmol) Methansulfonsäurechlorid zugetropft. Man rührt 2 Stunden bei Raumtemperatur, wäscht mit Wasser, trocknet über Magnesiumsulfat und engt im Vakuum ein. Der Rückstand wird in Dimethylformamid (DMF) aufgenommen und bei 0°C zu einer Suspension von 12,9 g (75 mmol) 4,6-Dimethoxypyrimidin-2-thiol und 8,4 g (100 mmol) Natriumhydrogencarbonat in 100 ml DMF getropft. Nach 2 Stunden Rühren bei Raumtemperatur und weiteren 2 Stunden bei 60°C gießt man auf 1 l Eiswasser und saugt den entstandenen Niederschlag ab. Nach Trocknen verbleiben 2,5 g eines weißen Pulvers.
Ausbeute: 31 % (Diastereomerengemisch 1:1).

Analog den obigen Beispielen wurden die in Tabelle 2 genannten Verbindungen hergestellt.

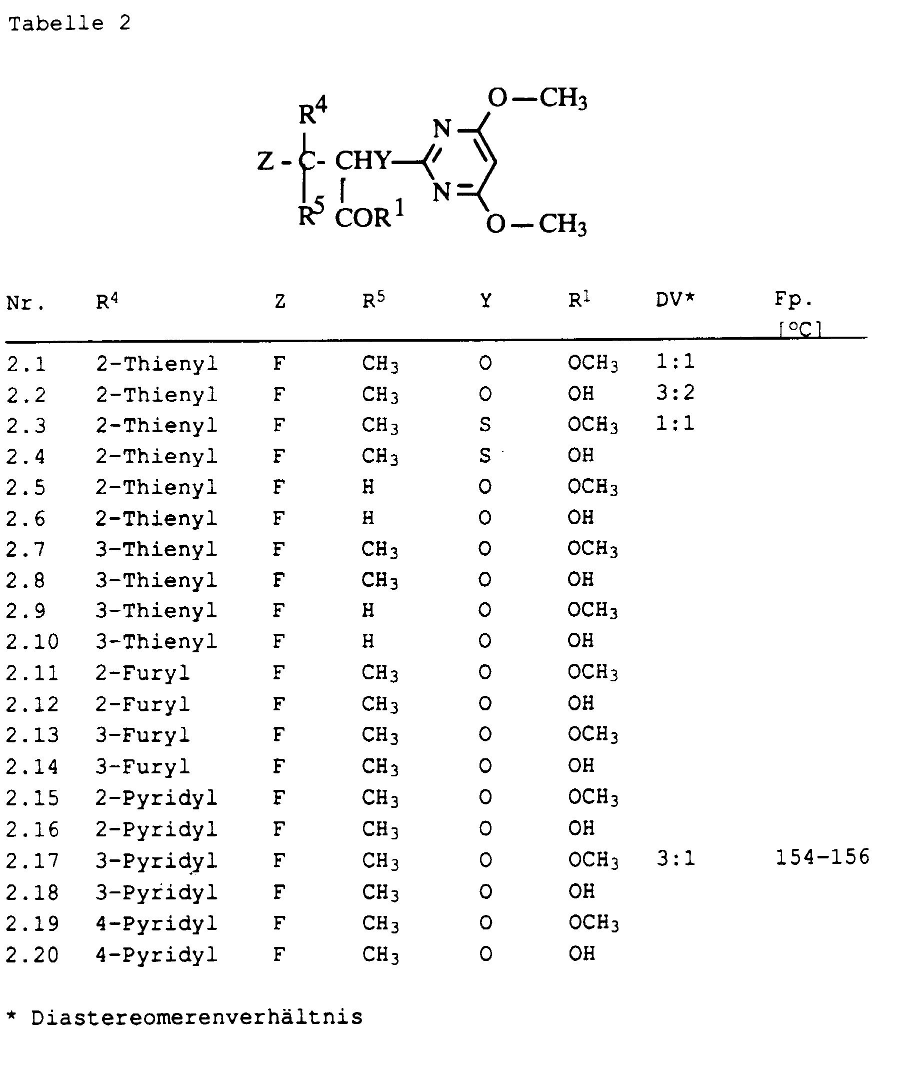

### Anwendungsbeispiele

Die herbizide Wirkung der 3-(Het)aryl-carbonsäurederivate der allgemeinen Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufanwendung werden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt.

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

## Patentansprüche

1. 3-Halogen-3-hetarylcarbonsäurederivate der allgemeinen Formel I 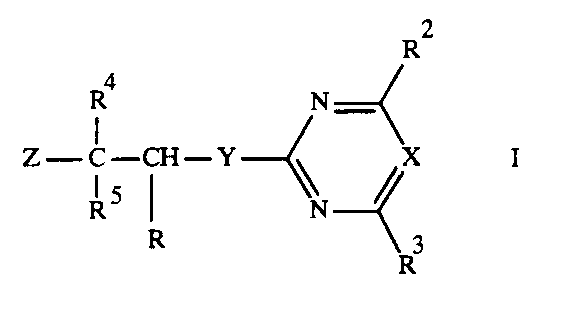 in der R eine Formylgruppe, eine Gruppe CO₂H oder einen zu COOH hydrolysierbaren Rest bedeutet und die übrigen Substituenten folgende Bedeutung haben:
R² Halogen, C₁-C₄-Alkyl, C₁-C₄-Halogenalkyl, C₁-C₄-Alkoxy, C₁-C₄-Halogenalkoxy oder C₁-C₄-Alkylthio;
X Stickstoff oder CR¹³, wobei R¹³ Wasserstoff bedeutet oder zusammen mit R³ eine 3- bis 4-gliedrige Alkylen- oder Alkenylenkette bildet, in der jeweils eine Methylengruppe durch Sauerstoff ersetzt ist;
R³ Halogen, C₁-C₄-Alkyl, C₁-C₄-Halogenalkyl, C₁-C₄-Alkoxy, C₁-C₄-Halogenalkoxy, C₁-C₄-Alkylthio oder R³ ist mit R¹³ wie oben angegeben zu einem 5- oder 6-gliedrigen Ring verknüpft;
R⁴ ein fünf- oder sechsgliedriger Heteroaromat, enthaltend ein bis drei Stickstoffatome und/oder ein Schwefel- oder Sauerstoffatom, welcher einen oder mehrere der folgenden Reste tragen kann: Halogen, Nitro, Cyano, Hydroxy, Mercapto, Amino, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino, Di-C₁-C₄-alkylamino, C₁-C₄-Alkylcarbonyl, C₁-C₄-Alkoxycarbonyl oder Phenyl;
R⁵ Wasserstoff, C₁-C₄-Alkyl, C₃-C₆-Alkenyl, C₃-C₆-Alkinyl, C₃-C₈-Cycloalkyl, C₁-C₄-Ralogenalkyl, C₁-C₄-Alkoxyalkyl, C₁-C₄-Alkylthioalkyl oder Phenyl;
Y Schwefel oder Sauerstoff oder eine Einfachbindung;
Z Halogen.

2. 3-Halogen-3-hetarylcarbonsäurederivate der allgemeinen Formel I gemäß Anspruch 1 in der R für eine Gruppe 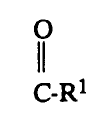 steht, wobei R¹ die folgende Bedeutung hat:
a) Wasserstoff;
b) eine Succinylimidoxygruppe;
c) ein über ein Stickstoffatom verknüpfter 5-gliedriger Heteroaromat, enthaltend zwei bis drei Stickstoffatome, welcher ein bis zwei Halogenatome und/oder ein bis zwei der folgenden Reste tragen kann:
C₁-C₄-Alkyl, C₁-C₄-Halogenalkyl, C₁-C₄-Alkoxy, C₁-C₄-Halogenalkoxy und/oder C₁-C₄-Alkylthio;
d) ein Rest -(O)ₘ-NR⁶R⁷,
in dem m für 0 oder 1 steht und R⁶ und R⁷, die gleich oder unterschiedlich sein können, die folgende Bedeutung haben:
Wasserstoff;
C₁-C₈-Alkyl, C₃-C₆-Alkenyl, C₃-C₆-Alkinyl, C₃-C₈-Cycloalkyl, wobei diese Reste jeweils ein bis fünf Halogenatome und/oder ein bis zwei der folgenden Gruppen tragen können: C₁-C₄-Alkoxy, C₃-C₆-Alkenyloxy, C₃-C₆-Alkinyloxy, C₁-C₄-Alkylthio, C₃-C₆-Alkenylthio, C₃-C₆-Alkinylthio, C₁-C₄-Halogenalkoxy, C₁-C₄-Alkylcarbonyl, C₃-C₆-Alkenylcarbonyl, C₃-C₆-Alkinylcarbonyl, C₁-C₄-Alkoxycarbonyl, C₃-C₆-Alkenyl oxycarbonyl, C₃-C₆-Alkinyloxycarbonyl, Di-C₁-C₄-alkylamino, C₃-C₈-Cycloalkyl, Phenyl, ein oder mehrfach durch Halogen, Nitro, Cyano, C₁-C₄-Alkyl, C₁-C₄-Ralogenalkyl, C₁-C₄-Alkoxy, C₁-C₄-Halogenalkoxy oder C₁-C₄-Alkylthio substituiertes Phenyl;
Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;
R⁶ und R⁷ gemeinsam eine zu einem Ring geschlossene, optionell substituierte C₄-C₇-Alkylenkette oder gemeinsam eine zu einem Ring geschlossene, optionell substituierte C₃-C₆-Alkylenkette mit einem Heteroatom, ausgewählt aus der Gruppe Sauerstoff, Schwefel oder Stickstoff;
e) R¹ ferner eine Gruppe 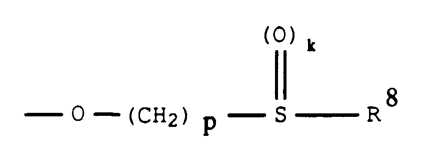 in der R⁸ für C₁-C₄-Alkyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl, C₁-C₄-Halogenalkyl, C₃-C₆-Alkenyl oder C₃-C₆-Alkinyl steht, p die Werte 1, 2, 3 oder 4 und k die Werte 0, 1 oder 2 annehmen;
f) einen Rest OR⁹, worin R⁹ bedeutet:
i) Wasserstoff, ein Alkalimetallkation, das Äquivalent eines Erdalkalimetallkations, das Ammoniumkation oder ein organisches Ammoniumion;
ii) eine C₃-C₈-Cycloalkylgruppe, welche ein bis drei C₁-C₄-Alkylreste tragen kann;
iii)eine C₁-C₈-Alkylgruppe, welche ein bis fünf Halogenatome und/oder einen der folgenden Reste tragen kann:
C₁-C₄-Alkoxy, C₁-C₄-Alkylthio, Cyano, C₁-C₄-Alkylcarbonyl, C₃-C₈-Cycloalkyl, C₁-C₄-Alkoxycarbonyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halo genalkoxy und/oder C₁-C₄-Alkylthio substituiertes Phenyl oder Phenoxy;
iv) eine C₁-C₈-Alkylgruppe, welche ein bis fünf Halogenatome tragen kann und einen der folgenden Reste trägt: ein 5-gliedriger Heteroaromat, enthaltend ein bis drei Stickstoffatome, oder ein 5-gliedriger Heteroaromat enthaltend ein Stickstoffatom und ein Sauerstoff- oder Schwefelatom, welche ein bis vier Halogenatome und/oder ein bis zwei der folgenden Reste tragen können: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
v) eine C₂-C₆-Alkylgruppe, welche in der 2-Position einen der folgenden Reste trägt: C₁-C₄-Alkoxyimino, C₃-C₆-Alkenyloxyimino, C₃-C₆-Halogenalkenyloxyimino oder Benzyloxyimino;
vi) eine C₃-C₆-Alkenyl- oder eine C₃-C₆-Alkinylgruppe, wobei diese Gruppen ihrerseits ein bis fünf Halogenatome tragen können;
vii) ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
viii) ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
ix) R⁹ ferner eine Gruppe 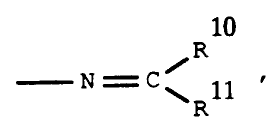 worin R¹⁰ und R¹¹, die gleich oder verschieden sind, bedeuten:
C₁-C₈-Alkyl, C₃-C₆-Alkenyl, C₃-C₆-Alkinyl, C₃-C₈-Cycloalkyl, wobei diese Reste einen C₁-C₄-Alkoxy-, C₁-C₄-Alkylthio und/oder einen Phenylrest tragen können;
Phenyl, das durch einen oder mehrere der folgenden Reste substituiert sein kann:
Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;
oder R¹⁰ und R¹¹ bilden gemeinsam eine C₃-C₁₂-Alkylenkette, welche ein bis drei C₁-C₄-Alkylgruppen tragen kann;
g) oder R¹ bildet einen Rest -NH-SO₂-R¹², in dem R¹² bedeutet:
C₁-C₄-Alkyl, C₃-C₆-Alkenyl, C₃-C₆-Alkinyl, C₃-C₈-Cycloalkyl, wobei diese Reste einen C₁-C₄-Alkoxy-, C₁-C₄-Alkylthio und/oder einen Phenylrest tragen können;
Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;

3. 3-Halogen-3-hetarylcarbonsäurederivate der Formel I gemäß Anspruch 1, in denen R⁵ Methyl, X CH, R² und R³ Methoxy bedeuten und Y, Z, R¹ und R⁴ die in Anspruch 1 genannte Bedeutung haben.

4. 3-Hetarylcarbonsäurederivate der Formel I gemäß den Anspruch 1, in denen Z Fluor, R⁵ Methyl, X CH, R² und R³ Methoxy bedeuten und Y, R¹ und R⁴ die in Anspruch 1 genannte Bedeutung haben.

5. Herbizides Mittel, enthaltend eine Verbindung der Formel I gemäß Anspruch 1 und übliche inerte Zusatzstoffe.

6. Verfahren zur Bekämpfung unerwünschten Pflanzenwuchses, dadurch gekennzeichnet, daß man eine herbizid wirksame Menge einer Verbindung der Formel I gemäß Anspruch 1 auf die Pflanzen oder deren Lebensraum einwirken läßt.

7. Mittel zur Beeinflussung des Pflanzenwachstums, enthaltend eine Verbindung der Formel I gemäß Anspruch 1 und übliche inerte Zusatzstoffe.

8. Verfahren zur Regulierung des Pflanzenwachstums, dadurch gekennzeichnet, daß man eine bioregulatorisch wirksame Menge einer Verbindung der Formel I gemäß Anspruch 1 auf die Pflanzen oder deren Lebensraum einwirken läßt.

9. Halogen-3-hetarylcarbonsäurederivate der allgemeinen Formel VI, 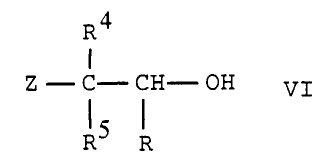 in der R, R⁴, R⁵ und Z die in Anspruch 1 genannte Bedeutung haben, ausgenommen die Verbindungen VI, in denen R⁴ unsubstituiertes Thienyl oder Furyl bedeutet.

10. Verfahren zur Herstellung von 3-Hetarylcarbonsäurederivaten der allgemeinen Formel VI gemäß Anspruch 9, dadurch gekennzeichnet, daß man Epoxide der allgemeinen Formel IV, 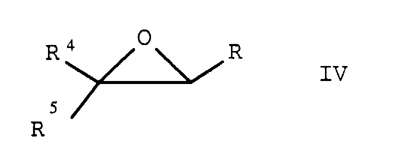 in der R, R⁴ und R⁵ die in Anspruch 1 genannte Bedeutung haben, mit Verbindungen der allgemeinen Formel V,
MZ V
in der Z für Halogen steht und M ein Alkalimetallkation, das Äquivalent eines Erdalkalimetallkations oder Wasserstoff bedeutet, gegebenenfalls in einem inerten Lösungsmittel und/ oder unter Zusatz eines geeigneten Katalysators, zur Reaktion bringt.

11. Verfahren zur Herstellung von 3-Halogen-3-hetarylcarbonsäurederivaten der allgemeinen Formel I gemäß Anspruch 1, wobei Y Sauerstoff bedeutet, dadurch gekennzeichnet, daß man 3-Halogen-3-hetarylcarbonsäurederivate der Formel VI, 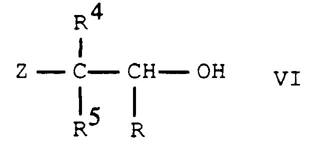 in der die Substituenten die in Anspruch 1 genannte Bedeutung haben, mit Verbindungen der allgemeinen Formel VII, 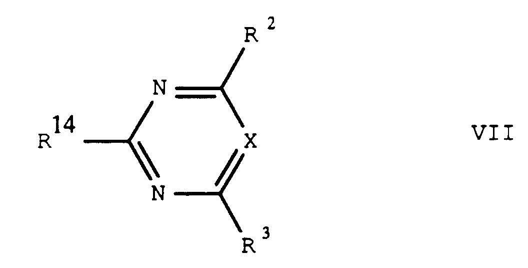 in der R¹⁴ Halogen oder R¹⁵SO₂- bedeutet, wobei R¹⁵ für C₁-C₄-Alkyl, C₁-C₄-Halogenalkyl oder Phenyl steht, in einem inerten Lösungsmittel in Gegenwart einer Base umsetzt.

12. Verfahren zur Herstellung von 3-Halogen-3-hetarylcarbonsäure-derivaten der allgemeinen Formel I gemäß Anspruch 1, wobei Y Schwefel bedeutet, dadurch gekennzeichnet, daß man 3-Halogen-3-hetarylcarbonsäurederivate der Formel VIII, 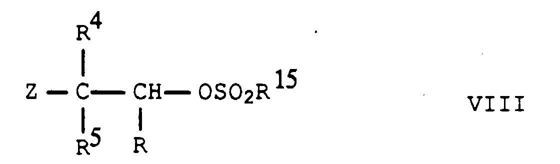 in der die Substituenten die in Anspruch 10 genannte Bedeutung haben, mit Verbindungen der allgemeinen Formel IX, 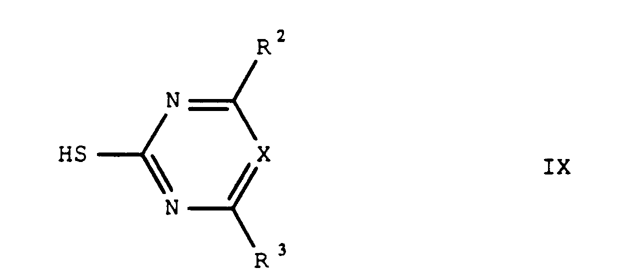 in der R², R³ und X die in Anspruch 1 genannte Bedeutung haben, zur Reaktion bringt.

## Claims

1. A 3-halo-3-hetarylcarboxylic acid derivative of the general formula I 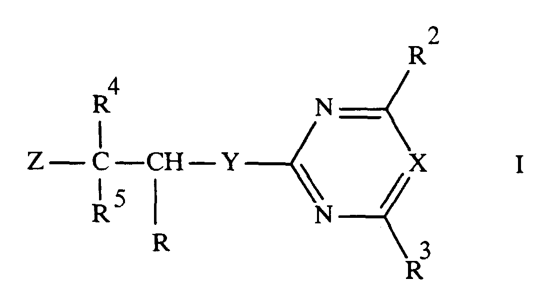 in which R is a formyl group, a group CO₂H or a radical which can be hydrolyzed to COOH, and the remaining substituents have the following meanings:
R² is halogen, C₁-C₄-alkyl, C₁-C₄-haloalkyl, C₁-C₄-alkoxy, C₁-C₄-haloalkoxy or C₁-C₄-alkylthio;
X is nitrogen or CR¹³, where R¹³ is hydrogen or together with R³ forms a 3- to 4-membered alkylene or alkenylene chain in which in each case one methylene group is replaced by oxygen;
R³ is halogen, C₁-C₄-alkyl, C₁-C₄-haloalkyl, C₁-C₄-alkoxy, C₁-C₄-haloalkoxy, C₁-C₄-alkylthio, or R³ is linked to R¹³ as mentioned above to form a 5- or 6-membered ring;
R⁴ is a five- or six-membered heteroaromatic ring which contains one to three nitrogen atoms and/or one sulfur or oxygen atom and which can have attached to it one or more of the following radicals: halogen, nitro, cyano, hydroxyl, mercapto, amino, C₁-C₄-alkyl, C₁-C₄-haloalkyl, C₁-C₄-alkoxy, C₁-C₄-haloalkoxy, C₁-C₄-alkylthio, C₁-C₄-alkylamino, di-C₁-C₄-alkylamino, C₁-C₄-alkylcarbonyl, C₁-C₄-alkoxycarbonyl or phenyl;
R⁵ is hydrogen, C₁-C₄-alkyl, C₃-C₆-alkenyl, C₃-C₆-alkynyl, C₃-C₈-cycloalkyl, C₁-C₄-haloalkyl, C₁-C₄-alkoxyalkyl, C₁-C₄-alkylthioalkyl or phenyl;
Y is sulfur or oxygen or a single bond; and
z is halogen.

2. A 3-halo-3-hetarylcarboxylic acid derivative of the general formula I as claimed in claim 1 where R is a group 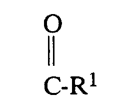 in which R¹ has the following meanings:
a) hydrogen;
b) a succinylimidoxy group;
c) a 5-membered heteroaromatic ring which is linked via a nitrogen atom, which contains two or three nitrogen atoms, and which can have attached to it one or two halogen atoms and/or one or two of the following radicals:
C₁-C₄-alkyl, C₁-C₄-haloalkyl, C₁-C₄-alkoxy, C₁-C₄-haloalkoxy and/or C₁-C₄-alkylthio;
d) a radical -(O)ₘ-NR⁶R⁷,
in which m is 0 or 1 and R⁶ and R⁷ can be identical or different and have the following meanings:
hydrogen;
C₁-C₈-alkyl, C₃-C₆-alkenyl, C₃-C₆-alkynyl, C₃-C₈-cycloalkyl, it being possible for these radicals to have attached to them in each case one to five halogen atoms and/or one or two of the following groups: C₁-C₄-alkoxy, C₃-C₆-alkenyloxy, C₃-C₆-alkynyloxy, C₁-C₄-alkylthio, C₃-C₆-alkenylthio, C₃-C₆-alkynylthio, C₁-C₄-haloalkoxy, C₁-C₄-alkylcarbonyl, C₃-C₆-alkenylcarbonyl, C₃-C₆-alkynylcarbonyl, C₁-C₄-alkoxycarbonyl, C₃-C₆-alkenyloxycarbonyl, C₃-C₆-alkynyloxycarbonyl, di-C₁-C₄-alkylamino, C₃-C₈-cycloalkyl, phenyl, or phenyl which is mono- or polysubstituted by halogen, nitro, cyano, C₁-C₄-alkyl, C₁-C₄-haloalkyl, C₁-C₄-alkoxy, C₁-C₄-haloalkoxy or C₁-C₄-alkylthio;
phenyl which can be substituted by one or more of the following radicals: halogen, nitro, cyano, C₁-C₄-alkyl, C₁-C₄-haloalkyl, C₁-C₄-alkoxy, C₁-C₄-haloalkoxy or C₁-C₄-alkylthio;
R⁶ and R⁷ together are a cyclized, substituted or unsubstituted C₄-C₇-alkylene chain or together are a cyclized, substituted or unsubstituted C₃-C₆-alkylene chain having one hetero atom selected from the group consisting of oxygen, sulfur or nitrogen;
e) R¹ is furthermore a group 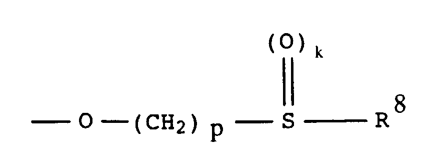 in which R⁸ is C₁-C₄-alkyl, phenyl, phenyl which is mono- or polysubstituted by halogen, nitro, cyano, C₁-C₄-alkyl, C₁-C₄-haloalkyl, C₁-C₄-alkoxy, C₁-C₄-haloalkoxy or C₁-C₄-alkylthio, or C₁-C₄-haloalkyl, C₃-C₆-alkenyl or C₃-C₆-alkynyl, p assumes the values 1, 2, 3 or 4 and k assumes the values 0, 1 or 2;
f) a radical OR⁹ where R⁹ is:
i) hydrogen, an alkali metal cation, the equivalent of an alkaline earth metal cation, the ammonium cation or an organic ammonium ion;
ii) a C₃-C₈-cycloalkyl group which can have attached to it one to three C₁-C₄-alkyl radicals;
iii) a C₁-C₈-alkyl group which can have attached to it one to five halogen atoms and/or one of the following radicals:
C₁-C₄-alkoxy, C₁-C₄-alkylthio, cyano, C₁-C₄-alkylcarbonyl, C₃-C₈-cycloalkyl, C₁-C₄-alkoxycarbonyl, phenyl, phenyl which is mono- or polysubstituted by halogen, nitro, cyano, C₁-C₄-alkyl, C₁-C₄-haloalkyl, C₁-C₄-alkoxy, C₁-C₄-haloalkoxy and/or C₁-C₄-alkylthio, or phenoxy;
iv) a C₁-C₈-alkyl group which can have attached to it one to five halogen atoms and which has attached to it one of the following radicals: a 5-membered heteroaromatic ring which contains one to three nitrogen atoms, or a 5-membered heteroaromatic ring which contains one nitrogen atom and one oxygen or sulfur atom, it being possible for the heteroaromatic rings to have attached to them one to four halogen atoms and/or one or two of the following radicals: nitro, cyano, C₁-C₄-alkyl, C₁-C₄-haloalkyl, C₁-C₄-alkoxy, C₁-C₄-haloalkoxy and/or C₁-C₄-alkylthio;
v) a C₂-C₆-alkyl group which has attached to it in the 2-position one of the following radicals: C₁-C₄-alkoxyimino, C₃-C₆-alkenyloxyimino, C₃-C₆-haloalkenyloxyimino or benzyloxyimino;
vi) a C₃-C₆-alkenyl or C₃-C₆-alkynyl group, it being possible for these groups, in turn, to have attached to them one to five halogen atoms;
vii) a phenyl radical which can have attached to it one to five halogen atoms and/or one to three of the following radicals: nitro, cyano, C₁-C₄-alkyl, C₁-C₄-haloalkyl, C₁-C₄-alkoxy, C₁-C₄-haloalkoxy and/or C₁-C₄-alkylthio;
viii) a 5-membered heteroaromatic ring which is linked via a nitrogen atom, which contains one to three nitrogen atoms, and which can have attached to it one or two halogen atoms and/or one or two of the following radicals: nitro, cyano, C₁-C₄-alkyl, C₁-C₄-haloalkyl, C₁-C₄-alkoxy, C₁-C₄-haloalkoxy and/or C₁-C₄-alkylthio;
ix) R⁹ is furthermore a group 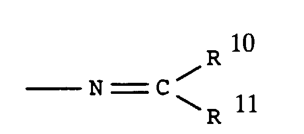 where R¹⁰ and R¹¹ are identical or different and are:
C₁-C₈-alkyl, C₃-C₆-alkenyl, C₃-C₆-alkynyl, C₃-C₈-cycloalkyl, it being possible for these radicals to have attached to them a C₁-C₄-alkoxy, C₁-C₄-alkylthio and/or phenyl radical;
phenyl which can be substituted by one or more of the following radicals:
halogen, nitro, cyano, C₁-C₄-alkyl, C₁-C₄-haloalkyl, C₁-C₄-alkoxy, C₁-C₄-haloalkoxy or C₁-C₄-alkylthio;
or R¹⁰ and R¹¹ together form a C₃-C₁₂-alkylene chain which can have attached to it one to three C₁-C₄-alkyl groups;
g) or R¹ forms a radical -NH-SO₂-R¹² where R¹² is:
C₁-C₄-alkyl, C₃-C₆-alkenyl, C₃-C₆-alkynyl, C₃-C₈-cycloalkyl, it being possible for these radicals to have attached to them a C₁-C₄-alkoxy, C₁-C₄-alkylthio and/or a phenyl radical;
phenyl which can be substituted by one or more of the following radicals: halogen, nitro, cyano, C₁-C₄-alkyl, C₁-C₄-haloalkyl, C₁-C₄-alkoxy, C₁-C₄-haloalkoxy or C₁-C₄-alkylthio.

3. A 3-halo-3-hetarylcarboxylic acid derivative of the formula I as claimed in claim 1, where R⁵ is methyl, X is CH, R² and R³ are methoxy and Y, Z, R¹ and R⁴ have the meanings given in claim 1.

4. A 3-halo-3-hetarylcarboxylic acid derivative of the formula I as claimed in claim 1, where Z is fluorine, R⁵ is methyl, X is CH, R² and R³ are methoxy and Y, R¹ and R⁴ have the meanings given in claim 1.

5. A herbicidal composition comprising a compound of the formula I as claimed in claim 1 and customary inert additives.

6. A method of controlling undesired plant growth, which comprises allowing a herbicidally active amount of a compound of the formula I as claimed in claim 1 to act on the plants or their environment.

7. A composition for influencing the growth of plants, comprising a compound of the formula I as claimed in claim 1 and customary inert additives.

8. A method of regulating the growth of plants, which comprises allowing a bioregulatory amount of a compound of the formula I as claimed in claim 1 to act on the plants or their environment.

9. A halo-3-hetarylcarboxylic acid derivative of the general formula VI 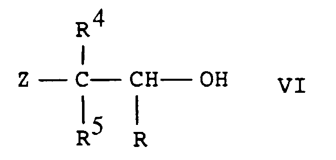 in which R, R⁴, R⁵ and Z have the meanings given in claim 1, excepting the compounds VI in which R⁴ is unsubstituted thienyl or furyl.

10. A process for the preparation of 3-hetarylcarboxylic acid derivatives of the general formula VI as claimed in claim 9, which comprises reacting epoxides of the general formula IV 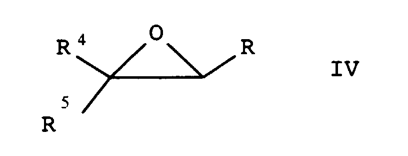 where R, R⁴ and R⁵ have the meanings given in claim 1 with compounds of the general formula V
MZ V
where Z is halogen and M is an alkali metal cation, the equivalent of an alkaline earth metal cation or hydrogen, in the presence or absence of an inert solvent and/or in the presence of a suitable catalyst.

11. A process for the preparation of 3-halo-3-hetarylcarboxylic acid derivatives of the general formula I as claimed in claim 1, where Y is oxygen, which comprises reacting 3-halo-3-hetarylcarboxylic acid derivatives of the formula VI 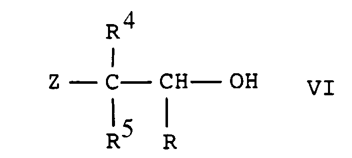 where the substituents have the meanings given in claim 1, with compounds of the general formula VII 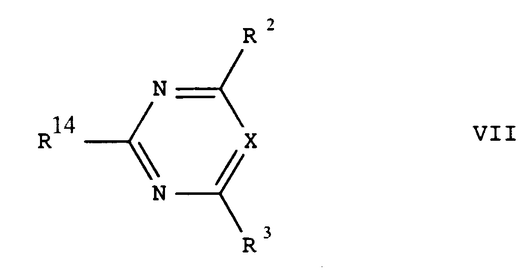 where R¹⁴ is halogen or R¹⁵SO₂-, R¹⁵ being C₁-C₄-alkyl, C₁-C₄-haloalkyl or phenyl, in an inert solvent in the presence of a base.

12. A process for the preparation of 3-halo-3-hetarylcarboxylic acid derivatives of the general formula I as claimed in claim 1, where Y is sulfur, which comprises reacting 3-halo-3-hetarylcarboxylic acid derivatives of the formula VIII 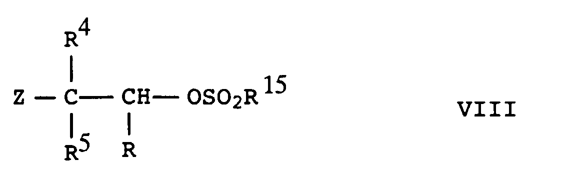 where the substituents have the meanings given in claim 10, with compounds of the general formula IX 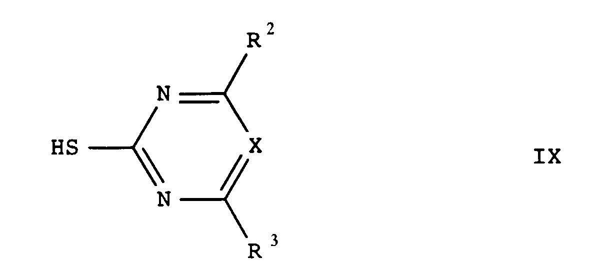 where R², R³ and X have the meanings given in claim 1.

## Revendications

1. Dérivés d'acides 3-halogéno-3-hétéroarylcarboxyliques de formule générale I 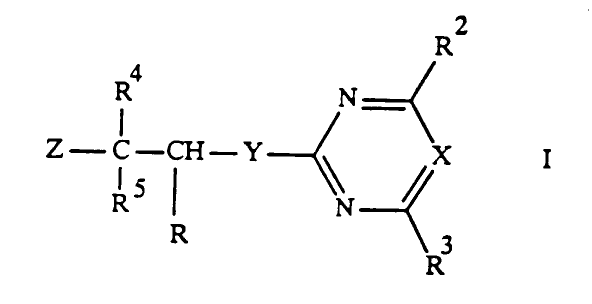 dans laquelle R représente un groupe formyle, un groupe CO₂H ou un groupe hydrolysable en un groupe COOH et les autres symboles ont les significations suivantes :
R² représente un halogène, un groupe alkyle en C1-C4, halogénoalkyle en C1-C4, alcoxy en C1-C4, halogénoalcoxy en C1-C4 ou alkylthio en C1-C4 ;
X représente l'azote ou CR¹³ dans lequel R¹³ représente l'hydrogène ou forme avec R³ une chaîne alkylène ou alcénylène de trois à quatre chaînons dans laquelle un groupe méthylène est remplacé par l'oxygène ;
R³ représente un halogène, un groupe alkyle en C1-C4, halogénoalkyle en C1-C4, alcoxy en C1-C4, halogénoalcoxy en C1-C4, alkylthio en C1-C4 ou bien R³ est relié avec R¹³ comme indiqué ci-dessus avec formation d'un cycle à cinq ou six chaînons ;
R⁴ représente un groupe hétéroaromatique à cinq ou six chaînons contenant un à trois atomes d'azote et/ou un atome de soufre ou d'oxygène et qui peut porter un ou plusieurs des substituants suivants : halogéno, nitro, cyano, hydroxy, mercapto, amino, alkyle en C1-C4, halogénoalkyle en C1-C4, alcoxy en C1-C4, halogénoalcoxy en C1-C4, alkylthio en C1-C4, alkylamino en C1-C4, di-(alkyle en C1-C4)amino, (alkyle en C1-C4)carbonyle, (alcoxy en C1-C4)carbonyle ou phényle ;
R⁵ représente l'hydrogène, un groupe alkyle en C1-C4, alcényle en C3-C6, alcynyle en C3-C6, cycloalkyle en C3-C8, halogénoalkyle en C1-C4, (alcoxy en C1-C4)alkyle, (alkyle en C1-C4)thioalkyle ou phényle ;
Y représente le soufre ou l'oxygène ou une liaison simple et
R représente un halogène.

2. Dérivés d'acides 3-halogéno-3-hétéroarylcarboxyliques de formule générale I selon la revendication 1, dans laquelle R représente un groupe 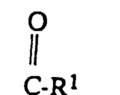 dans lequel R¹ a les significations suivantes :
a) l'hydrogène ;
b) un groupe succinylimidoxy ;
c) un groupe hétéroaromatique à cinq chaînons relié par l'intermédiaire d'un atome d'azote, qui contient deux à trois atomes d'azote et peut porter un à deux atomes d'halogènes et/ou un à deux des substituants suivants :
alkyle en C1-C4, halogénoalkyle en C1-C4, alcoxy en C1-C4, halogénoalcoxy en C1-C4 et/ou alkylthio en C1-C4 ;
d) un groupe -(O)ₘ-NR⁶R⁷,
dans lequel m est égal à 0 ou 1 et R⁶ et R⁷, ayant des significations identiques ou différentes, représentent chacun : l'hydrogène ;
un groupe alkyle en C1-C8, alcényle en C3-C6, alcynyle en C3-C6, cycloalkyle en C3-C8, chacun de ces groupes pouvant porter un à cinq atomes d'halogènes et/ou un à deux des substituants suivants : alcoxy en C1-C4, alcényloxy en C3-C6, alcynyloxy en C3-C6, alkylthio en C1-C4, alcénylthio en C3-C6, alcynylthio en C3-C6, halogénoalcoxy en C1-C4, (alkyle en C1-C4)carbonyle, (alcényle en C3-C6)carbonyle, (alcynyle en C3-C6)carbonyle, (alcoxy en C1-C4)carbonyle, (alcényle en C3-C6)oxycarbonyle, (alcynyle en C3-C6)oxycarbonyle, di-(alkyle en C1-C4)amino, cycloalkyle en C3-C8, phényle, phényle portant lui-même un ou plusieurs substituants halogéno, nitro, cyano, alkyle en C1-C4, halogénoalkyle en C1-C4, alcoxy en C1-C4, halogénoalcoxy en C1-C4 ou alkylthio en C1-C4 ;
un groupe phényle qui peut porter un ou plusieurs des substituants suivants : halogéno, nitro, cyano, alkyle en C1-C4, halogénoalkyle en C1-C4, alcoxy en C1-C4, halogénoalcoxy en C1-C4 ou alkylthio en C1-C4 ;
R⁶ et R⁷ représentent ensemble une chaîne alkylène en C4-C7 éventuellement substituée et fermée en un cycle ou bien une chaîne alkylène en C3-C6 contenant un hétéroatome choisi parmi l'oxygène, le soufre ou l'azote, éventuellement substituée et fermée en un cycle ;
e) R¹ peut en outre représenter un groupe 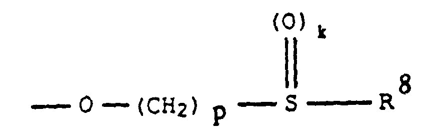 dans lequel R⁸ représente un groupe alkyle en C1-C4, phényle, phényle portant un ou plusieurs substituants halogéno, nitro, cyano, alkyle en C1-C4, halogénoalkyle en C1-C4, alcoxy en C1-C4, halogénoalcoxy en C1-C4 ou alkylthio en C1-C4, un groupe halogéno-alkyle en C1-C4, alcényle en C3-C6 ou alcynyle en C3-C6, p est égal à 1, 2, 3 ou 4 et k à 0, 1 ou 2 ;
f) un groupe OR⁹ dans lequel R⁹ représente :
i) l'hydrogène, un cation de métal alcalin, un équivalent d'un cation de métal alcalino-terreux, le cation ammonium ou un ion ammonium organique ;
ii) un groupe cycloalkyle en C3-C8 qui peut porter un à trois groupes alkyle en C1-C4 ;
iii) un groupe alkyle en C1-C8 qui peut porter un à cinq atomes d'halogènes et/ou un des substituants suivants : alcoxy en C1-C4, alkylthio en C1-C4, cyano, (alkyle en C1-C4)carbonyle, cycloalkyle en C3-C8, (alcoxy en C1-C4)carbonyle, phényle, phényle ou phénoxy portant un ou plusieurs substituants halogéno, .nitro, cyano, alkyle en C1-C4, halogénoalkyle en C1-C4, alcoxy en C1-C4, halogénoalcoxy en C1-C4 et/ou alkylthio en C1-C4 ;
iv) un groupe alkyle en C1-C8 qui peut porter un à cinq atomes d'halogènes et porte l'un des substituants suivants : un groupe hétéroaromatique à cinq chaînons contenant un à trois atomes d'azote ou un groupe hétéroaromatique à cinq chaînons contenant un atome d'azote et un atome d'oxygène ou de soufre, qui peuvent porter un à quatre atomes d'halogènes et/ou un à deux des substituants suivants : nitro, cyano, alkyle en C1-C4, halogénoalkyle en C1-C4, alcoxy en C1-C4, halogénoalcoxy en C1-C4 et/ou alkylthio en C1-C4 ;
v) un groupe alkyle en C2-C6 portant en position 2 l'un des substituants suivants : alcoxyimino en C1-C4, alcényloxyimino en C3-C6, halogénoalcényloxyimino en C3-C6 ou benzyloxyimino ;
vi) un groupe alcényle en C3-C6 ou alcynyle en C3-C6 pouvant chacun porter un à cinq atomes d'halogènes ;
vii) un groupe phényle qui peut porter un à cinq atomes d'halogènes et/ou un à trois des substituants suivants : nitro, cyano, alkyle en C1-C4, halogénoalkyle en C1-C4, alcoxy en C1-C4, halogénoalcoxy en C1-C4 et/ou alkylthio en C1-C4 ;
viii) un groupe hétéroaromatique à cinq chaînons relié par un atome d'azote, contenant un à trois atomes d'azote et qui peut porter un à deux atomes d'halogènes et/ou un à deux des substituants suivants : nitro, cyano, alkyle en C1-C4, halogénoalkyle en C1-C4, alcoxy en C1-C4, halogénoalcoxy en C1-C4 et/ou alkylthio en C1-C4 ;
ix) R⁹ peut en outre représenter un groupe 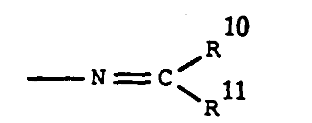
dans lequel R¹⁰ et R¹¹, ayant des significations identiques ou différentes, représentent chacun : un groupe alkyle en C1-C8, alcényle en C3-C6, alcynyle en C3-C6, cycloalkyle en C3-C8, lesquels peuvent porter un groupe alcoxy en C1-C4, alkylthio en C1-C4 et/ou phényle ;
un groupe phényle qui peut porter un ou plusieurs des substituants suivants :
halogéno, nitro, cyano, alkyle en C1-C4, halogénoalkyle en C1-C4, alcoxy en C1-C4, halogénoalcoxy en Cl-C4 ou alkylthio en C1-C4 ;
ou bien R¹⁰ et R¹¹ forment ensemble une chaîne alkylène en C3-C12 qui peut porter un à trois groupes alkyle en C1-C4 ;
g) ou bien R¹ représente un groupe -NH-SO₂-R¹² dans lequel R¹² représente :
un groupe alkyle en C1-C4, alcényle en C3-C6, alcynyle en C3-C6, cycloalkyle en C3-C8, lesquels peuvent porter un groupe alcoxy en C1-C4, alkylthio en C1-C4 et/ou phényle ;
un groupe phényle qui peut porter un ou plusieurs des substituants suivants : halogéno, nitro, cyano, alkyle en C1-C4, halogénoalkyle en C1-C4, alcoxy en C1-C4, halogénoalcoxy en C1-C4 ou alkylthio en C1-C4.

3. Dérivés d'acides 3-halogéno-3-hétéroarylcarboxyliques de formule I selon la revendication 1 dans laquelle R⁵ représente un groupe méthyle, X représente CH, R² et R³ des groupes méthoxy et Y, Z, R¹ et R⁴ ont les significations indiquées dans la revendication 1.

4. Dérivés d'acides 3-hétéroarylcarboxyliques de formule I selon la revendication 1, dans laquelle Z représente le fluor, R⁵ un groupe méthyle, X représente CH, R² et R³ des groupes méthoxy et Y, Z, R¹ et R⁴ ont les significations indiquées dans la revendication 1.

5. Produit herbicide contenant un composé de formule I selon la revendication 1 et des additifs usuels inertes.

6. Procédé pour combattre les croissances de végétaux indésirables, caractérisé par le fait que l'on fait agir une quantité herbicide efficace d'un composé de formule I selon la revendication 1 sur les végétaux ou leur habitat.

7. Produit pour agir sur la croissance des végétaux, contenant un composé de formule I selon la revendication 1 et des additifs usuels inertes.

8. Procédé pour la régulation de la croissance des végétaux, caractérisé par le fait que l'on fait agir une quantité biorégulatrice efficace d'un composé de formule I selon la revendication 1 sur les végétaux ou leur habitat.

9. Dérivés d'acides halogéno-3-hétéroarylcarboxyliques de formule générale VI, 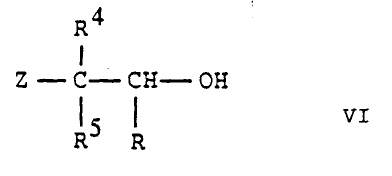 dans laquelle R, R⁴, R⁵ et Z ont les significations indiquées dans la revendication 1, à l'exception des composés de formule VI dans laquelle R⁴ représente un groupe thiényle ou furyle non substitué.

10. Procédé de préparation des dérivés d'acides 3-hétéroarylcarboxyliques de formule générale VI selon la revendication 9, caractérisé par le fait que l'on fait réagir des époxydes de formule générale IV 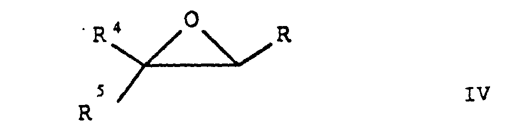 dans laquelle R, R⁴ et R⁵ ont les significations indiquées dans la revendication 1, avec des composés de formule générale V
MZ V
dans laquelle Z représente un halogène et M un cation de métal alcalin, un équivalent d'un cation de métal alcalino-terreux ou l'hydrogène, le cas échéant dans un solvant inerte et/ou avec adjonction d'un catalyseur approprié.

11. Procédé de préparation des dérivés d'acides 3-halogéno-3-hétéroarylcarboxyliques de formule générale I selon la revendication 1, dans laquelle Y représente l'oxygène, caractérisé par le fait que l'on fait réagir des dérivés d'acides 3-halogéno-3-hétéroarylcarboxyliques de formule VI 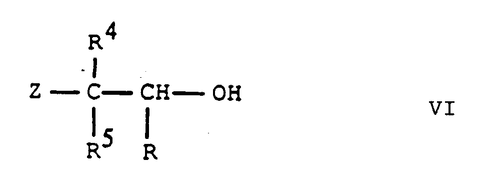 dans laquelle les symboles ont les significations indiquées dans la revendication 1, avec des composés de formule générale VII 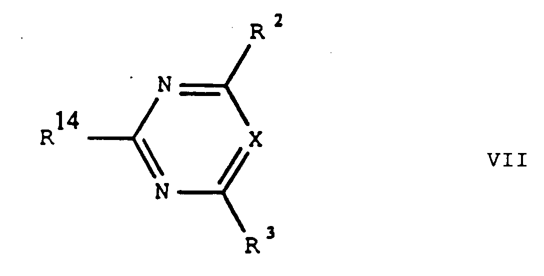 dans laquelle R¹⁴ représente un halogène ou un groupe R¹⁵SO₂- dans lequel R¹⁵ représente un groupe alkyle en C1-C4, halogénoalkyle en C1-C4 ou phényle, dans un solvant inerte et en présence d'une base.

12. Procédé de préparation des dérivés d'acides 3-halogéno-3-hétéroarylcarboxyliques de formule générale I selon la revendication 1 dans laquelle Y représente le soufre, caractérisé par le fait que l'on fait réagir des dérivés d'acides 3-halogéno-3-hétéroarylcarboxyliques de formule VIII 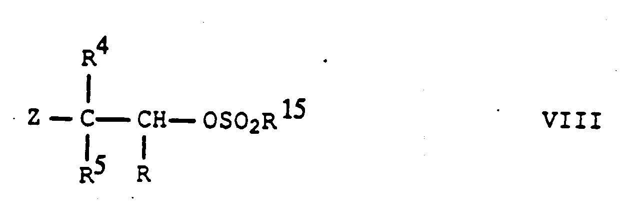 dans laquelle les symboles ont les significations indiquées dans la revendication 10, avec des composés de formule générale IX 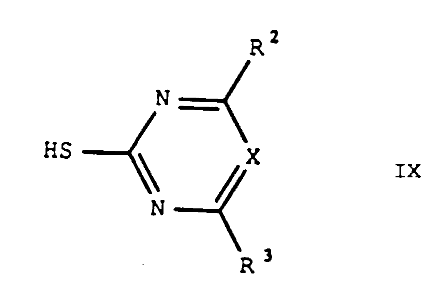 dans laquelle R², R³ et X ont les significations indiquées dans la revendication 1.
